Europäisches Patentamt

European Patent Office

Office européen des brevets

(19)

(11) Veröffentlichungsnummer: **0 163 045**
**B1**

(12) ## EUROPÄISCHE PATENTSCHRIFT

(45) Veröffentlichungstag der Patentschrift: 29.08.90

(51) Int. Cl.⁵: **B 32 B 15/08**

(21) Anmeldenummer: **85103619.4**

(22) Anmeldetag: **27.03.85**

(54) Mittel zur Speicherung und Verteilung von Wärme.

(30) Priorität: 30.05.84 DE 3420121
20.06.84 DE 3422783
27.08.84 DE 3431474
30.10.84 DE 3439727

(43) Veröffentlichungstag der Anmeldung:
04.12.85 Patentblatt 85/49

(45) Bekanntmachung des Hinweises auf die
Patenterteilung:
29.08.90 Patentblatt 90/35

(84) Benannte Vertragsstaaten:
AT BE CH DE FR GB IT LI LU NL SE

(56) Entgegenhaltungen:
CH-A- 370 192
DE-U-7 627 371
DE-U-8 130 561
GB-A- 742 787
GB-A-1 212 291
US-A-2 572 670

(73) Patentinhaber: Indentor AG
Oberlindenberg 191
CH-9427 Wolfhalden (CH)

(72) Erfinder: Latzke, Arno Walter
Mühltobel 946
CH-9429 Zelg-Wolfhalden (CH)

(74) Vertreter: Werner, Hans-Karsten, Dr. et al
Deichmannhaus am Hauptbahnhof
D-5000 Köln 1 (DE)

EP 0 163 045 B1

Courier Press, Leamington Spa, England.

**Beschreibung**

Gegenstand der vorliegenden Erfindung sind Mittel zur Wärmeisolierung und zur Speicherung und Verteilung von Körperwärme auf Flächen der Außenhaut.

Eine Reihe von Schmerzen, Verkrampfungen und krankhafter Störungen beruhen auf einer ungenügenden oder ungleichmäßigen Durchblutung der Außenhaut. In vielen Fällen läßt sich durch gleichmäßige Einwirkung von Wärme und ggf. auch Wärme und Feuchtigkeit Linderung, Besserung oder sogar Heilung erzielen. Die Behandlung mit Infrarot-Strahlen, Wärmflaschen, Heizkissen, heißen Packungen und Umschlägen ist stets mit erheblichem Aufwand verbunden und daher nicht immer und ohne weiteres durchführbar. Auch kalte Füsse beruhen auf derartigen Effekten und lassen sich mit herkömmlichen Mitteln nur ungenügend oder unbequem behandeln.

Die GB-A-742787 beschreibt eine laminierte Einlegesohle für Schuhe, die ein hitzeregulierendes Laminat sowie ein oder mehrere Laminate aus flexiblem Material auf beiden Seiten des hitzereflektierenden Laminats enthält. Eines der äußeren Laminate besteht aus Latexschaumstoff.

Die Erfindung hat sich zunächst die Aufgabe gestellt, die lokalen Unterkühlungen und die ungenügende ungleichmäßige Durchblutung von Hautflächen in einfacher Weise zu behandeln. Dabei soll das Mittel einfach anzubringen sein, beim Tragen möglichst wenig stören, leicht entfernbar sein und obendrein technisch wenig aufwendig und preiswert sein.

Die Erfindung hat sich weiterhin die Aufgabe gestellt, verbesserte Mittel zur generellen Wärmeisolierung sowie zur Speicherung und Verteilung von Wärme, insbesondere von Körperwärme auf Flächen der Außenhaut zu entwickeln, die aus dickeren Schaumstoffschichten bestehen.

Derartige dickere Schaumstoffschichten führen daher wiederum zu dem bekannten lokalen Wärmestau. Für gewisse Anwendungen der neuen Mittel zur Speicherung und Verteilung von Wärme auf Flächen der Außenhaut ist es jedoch wünschenswert, dickere Schaumstoffschichten zu verwenden, da diese zu einer besseren Polsterung und Abfederung bei Druckbelastung führen.

Diese Aufgabe wird gelöst durch Mittel bestehend der Reihe nach aus

a) einer 0,8 bis 8 mm starken geschlossenporigen PE-Schaumstoffschicht (1);

b) einer flexiblen, wärmeleitenden Folie aus Aluminium (2) und

c) einer 0,8 bis 8 mm starken geschlossenporigen PE-Schaumstoffschicht (3), wobei die Schicht (1) mit durchblutungsfördernden Substanzen getränkt ist.

Die erfindungsgemäßen Mittel können beispielsweise bei kalten Füssen in die Form von Einlegesohlen gebracht werden und in üblichem Schuhwerk auf der Außenhaut der Fußsohle getragen werden. Zur Befestigung der erfindungsgemäßen Mittel auf anderen Körperteilen werden diese vorzugsweise mittels eines selbstklebenden Pflasters auf dem Körper befestigt. Anstelle des selbstklebenden Pflasters können sie selbstverständlich auch mit Hilfe einer Stoffbinde oder eines Strumpfes befestigt werden.

Eine weitere bevorzugte Ausführungsform weist auf der äußeren Formschicht zusätzlich einen Klettverschluß auf. Die so ausgestalteten erfindungsgemäßen Mittel lassen sich in besonders einfacher Weise mittels elastischer Binden mit Klettverschluß am Körper befestigen.

Schließlich ist es möglich, auf eine oder beide Schaumstoffschichten eine hautverträgliche, selbstklebende Schicht aufzubringen, die durch eine abziehbare Schutzfolie abgedeckt ist. Vor der Benutzung wird eine Schutzfolie abgezogen und das Mittel direkt auf die Haut geklebt. Sofern beide Schaumstoffschichten eine selbstklebende Schicht aufweisen, kann das Mittel zweimal verwendet werden. Gewünschtenfalls können sowohl die der Haut zugewandte Seite der PE-Schaumstoffschicht oder auch beide Schaumstoffschichten mit einer hautverträglichen Stoffschicht überzogen sein. Diese Stoffschicht ist in der Lage Wärme zu leiten und Feuchtigkeit aufzunehmen. Sofern sie mit dem erfindungsgemäßen Mittel fest verklebt ist, kann sie auch zur weiteren mechanischen Stabilisierung des Schaumstoffes beitragen. Als Stoffe kommen insbesondere solche aus Baumwolle in Frage, Flanell und Strickgewebe, sowie selbstklebene Vliese wie das Produkt Fixomull Stretch der Beiersdorf AG, Hamburg sind besonders gut geeignet.

Die erfindungsgemäßen Mittel sind insgesamt preiswert, einfach und angenehm zu tragen. Sämtliche Ausführungsformen des erfindungsgemäßen Mittels führen zu einer ausgezeichneten Speicherung und Verteilung der sonst an die Außenwelt abgegebenen Körperwärme auf die unter ihnen liegenden Flächen der Außenhaut. Die 0,8 bis 8 mm starken PE-Schaumstoffschichten sind wegen der guten Hautverträglichkeit von polyethylen geeignet, die abgegebene Wärme aber auch die abgegebene Feuchtigkeit zu speichern. Die flexiblen, wärmeleitfähigen Folien aus Aluminium in der Mitte führen zu einer gleichmäßigen Verteilung der Wärme innerhalb des Mittels, so daß schlecht durchblutete Hautpartien durch besser durchblutete Hautpartien beheizt werden. Die weitere 0,8 bis 8 mm starke, flexible PE-Schaumstoffschicht führt zu einer Wärmeisolierung der Folie gegen die Umwelt und verhindert die unerwünschte Abstrahlung der Wärme aus der Aluminiumschicht.

Die flexible, wärmeleitfähige Folie aus Aluminium hat den weiteren Vorteil, daß das erfindungsgemäße Mittel insgesamt stabiler und strapazierfähiger wird und nicht mehr so leicht reißt oder bricht. Die Aluminiumfolien weisen im allgemeinen Stärken von 0,08 bis 0,3 mm auf. Schwächere Folien sind mechanisch nicht stabil genug und führen auch zu einer schlechteren Ableitung der Wärme aus wärmeren Bezirken in kältere. Dickere Aluminiumfolien versteifen das erfindungsgemäße Mittel so sehr, daß es sich nicht mehr ohne weiteres überall an der Haut anschmiegt.

Die Aluminiumfolien wirken auch feuchtigkeitsisolierend. Es ist daher auch möglich, mit diesen Mitteln die abgedeckten Hautflächen einer feuchten Wärmebehandlung zu unterziehen.

Bei der Ausgestaltung der erfindungsgemäßen Mittel als Einlegesohlen führt die Verwendung von Aluminiumfolien ebenfalls zu einer Feuchtigkeitsisolierung, so daß einerseits durch die Einlegesohle keine Feuchtigkeit an den Fuß gelangen kann, andererseits jedoch auch gebildeter Fußschweiß nicht durch die Einlegesohle entweichen kann.

Die Verklebung der Folie aus Aluminium mit den beiden PE-Schaumstoffschichten erfolgt in an sich bekannter Weise mittels üblicher Klebstoffe, die sowohl mit dem verwendeten Kunststoff als auch mit dem verwendeten Aluminium gut haftende Verbindungen geben. Fertigungstechnisch besonders bevorzugt ist die Verwendung von selbstklebenden Schaumstoffbahnen, die mit den Aluminiumfolien verklebt werden.

Die Stärke der beiden PE-Schaumstoffschichten kann man von dem jeweiligen Verwendungszweck abhängig machen. Bei Einlegesohlen führen dickere PE-Schaumstoffschichten zu einer Federung der Fußsohle und zu einem oftmals zusätzlich erwünschten Massageeffekt.

Für erfindungsgemäße Mittel zur Behandlung von Nacken- und Schulterpartien werden dünnere Ausführungsformen bevorzugt, da sie weniger auftragen und dadurch weniger optisch stören. Für diesen Zweck sind weiterhin die Ausführungsformen mit einer selbstklebenden Schicht vorteilhaft einsetzbar. An Armen und Beinen wiederum werden Ausführungsformen bevorzugt, die sich in einfacher Weise mittels Binden, Strümpfen oder Klettverschlußbinden befestigen lassen und dadurch angenehm beim Tragen sind.

Der erfindungsgemäß verwendete PE-Schaumstoff ist nicht nur hautverträglich sondern auch wasserfest, so daß man ihn aus hygienischen Gründen nach dem Tragen mit heißem Wasser und einem Detergenz reinigen und wieder verwenden kann. Das Gleiche gilt für die Einlegesohlen, die man zur Beseitigung des daran abgelagerten Schweißes ohne weiteres auswaschen und wiederverwenden kann. Auch hierbei macht sich die verstärkende und stabilisierende Wirkung der Folie aus Aluminium sehr positiv bemerkbar, da die Schaumstoffschichten in einer Stärke von 0,8 bis 8 mm oder auch zusammen 1,6 bis 16 mm beim Reinigen leicht einreißen oder brechen würden. Bei Einlegesohlen kann selbstverständlich auch eine zusätzliche Abdeckung der Schaumstoffschichten mit Textilgewebe erfolgen.

Ein besonderer Vortel der Ausbildung des erfindungsgemäßen Mittels als Einlegesohlen ist, daß sie sich sowohl an die Fußkonturen als auch an die Konturen im Schuh gut anformen und dadurch einen optimalen und direkten Kontakt zwischen Sohle und Fuß herstellen.

Da auch die äußere Schaumstoffschicht aus polyethylen ist, kann das erfindungsgemäße Mittel auch von beiden Seiten nacheinander benutzt werden, so daß die Reinigung seltener nötig ist.

Die erfindungsgemäßen Mittel können selbstverständlich nicht nur auf der menschlichen Haut sondern auch bei Tieren angewendet werden, wobei bei behaarten Hautteilen und Fell vorzugsweise die Ausführungsformen mit Binden, Strümpfen oder Klettverschlüssen zur Anwendung kommen.

Je nach Anwendungszweck können dabei sowohl das Raumgewicht des geschlossenporigen PE-Schaumes als auch die Foliendicke des Aluminiums variiert werden. Auf alle Fälle läßt sich das erfindungsgemäße Material außerordentlich preiswert herstellen, aufrollen, lagern, transportieren und anschließend verarbeiten. Auch im verarbeiteten Zustand weist es exzellent mechanische Eigenschaften auf, so daß es langfristig für die gewünschte Wärmeisolierung sorgt.

Das Aufkleben der PE-Schaume auf die Aluminiumfolien erfolgt vorzugsweise durch Heißkleber oder durch flammkaschieren. Prinzipiell können aber auch alle anderen auf Aluminium und Polyethylen haftenden Klebstoffe verwendet werden.

Sofern eine Belüftung nötig ist, wird man das erfindungsgemäße Mittel perforieren. Die Lochgrößen werden dabei vorzugsweise in Abständen von 10 bis 20 mm angebracht. Die Lochgrößen liegen dann wiederum vorzugsweise im Bereich zwischen 1 und 5 mm Durchmesser.

Die Raumgewichte der verwendeten PE-Schaumstoffe können in weiten Grenzen variiert werden. Sie können zwischen 20 und 150 kg/m$^3$ liegen. Für Einlegesohlen haben sich Raumdichten zwischen 50 und 120 kg/m$^3$ besonders bewährt.

Insbesondere wenn das erfindungsgemäße Mittel als Einlegesohle oder integrierter Bestandteil einer Schuhsohle verwendet werden soll, hat es sich als besonders zweckmäßig erwiesen, angrenzend an die PE-Schaumstoffschicht (3) zusätzlich eine flexible, wärmeleitfähige Metallschicht (2') und eine weitere 0,8 bis 4 mm starke flexible Schaumstoffschicht (3') aus geschlossenporigem Polyethylenschaumstoff anzubringen.

Derartige, Einlegesohlen oder Bestandteile von Sohlen eines Schuhes weisen folgende wesentliche Vorteile auf:

1. Man erhält eine gleichmäßige, konstante und sich schnell angleichende Oberflächentemperatur der Fußkontaktfläche. Die von der Fußkontaktfläche abgegebene Wärme wird optimal gespeichert und führt somit zu nachhaltig und gleichmäßig warmen Füßen.

2. Überraschenderweise führt die gleichmäßige Erwärmung der Fußsohle zu einer verminderten Schweißbildung, so daß die Fußkontaktfläche einen niedrigen Feuchtigkeitsgrad aufweist.

3. Erfindungsgemäße Einlegesohlen weisen eine der Fußform entsprechende Verformung auf, die als ausgesprochen angenehm empfunden wird.

4. Die Sohlendicke ist erstaunlich gering, weist jedoch Wirkungen auf, die bisher nicht einmal durch wesentlich stärkere Materialien erzielt werden konnten.

Dies beruht insbesondere darauf, daß erfindungsgemäß erstmals ein guter Wärmeleiter mit einem guten Wärmeisolator kombiniert wird. Die Kombination dieser beiden verschiedenartigen Werkstoffe ist vom Fachmann bisher nie in Erwägung gezogen worden, da er zur Erzielung einer guten Wärmeisolierung nur Wärmeisolatoren verwendet hat und die Verwendung von gut wärmeleitfähigen Werkstoffen vermieden hat. Die paradox erscheinende Kombination bewirkt erstmals nicht nur eine gute Wärmespeicherung, sondern gleichzeitig eine gute flächenmäßige Verteilung der Wärme. Dadurch werden lokale Überhitzungen und lokale Unterkühlungen vermieden bzw. rasch ausgeglichen. Bekanntlich sind jedoch derartige lokale Überhitzungen und lokale Unterkühlungen Ursache von Schmerzen, Verspannungen, lokaler Schweißbildung und den sogenannten "brennenden" Füßen. Durch den Einbau der erfindungsgemäßen Mittel in Schuhsohlen kann auch erstmals das Problem der seit langem gesuchten optimalen Schuhsohle gelöst werden. Alle bisher verwendeten Materialien haben entweder ungenügende Wärmeisolierung aufgewiesen und führten daher zu kalten Füßen, oder aber isolierten die Wärme so gut, daß es zu lokalen Überhitzungen, Schweißfüßen und brennenden Füßen führte.

Da für Einlegesohlen und Sohlen geschlossenporiger Polyethylenschaum eingesetzt wird, kann sich dieser Schaumstoff nicht mit Wasser oder Schweiß füllen, und zwar nicht einmal von den Schnittkanten her. Die erfindungsgemäßen Mittel werden daher weder von innerer noch von äußerer Feuchtigkeit in ihrer Wirkung beeinträchtigt. Die verwendeten Materialien werden daher auch nicht von Mikroorganismen, wie Bakterien und Pilzen, befallen, so daß sie geruchsneutral bleiben, und keine Gerüche binden. Sie werden auch nicht durch Seifenwasser oder Waschmaschinen beschädigt und können daher gewünschtenfalls in einfacher Weise gereinigt werden. Sie entsprechen daher den heutigen hygienischen Anforderungen an Einlegesohlen und Sohlen.

Durch geeignete Wahl der Raumdichten der verwendeten Schaumstoffe läßt sich eine gewisse bleibende Verformung erzielen, die zu einer natürlichen Anpassung der Einlegesohle bzw. der Sohle an die individuelle Fußfläche gewährleistet. Dies führt zu einem optimalen Tragkomfort und wird als besonders angenehm empfunden.

Durch die Verwendung von 2 flexiblen, wärmeleitfähigen Folïe aus Aluminium (2) und (2') und die daran angrenzenden PE-Schaumstoffschichten (3) und (3') entstehen 2 "Wärmekammern" mit optimalen Isolierung und optimaler Verteilung der Wärme bzw. Abschirmung der Kälte durch die Schuhsohle. Messungen an derartigen erfindungsgemäßen Einlegesohlen haben gezeigt, daß auch bei höheren Temperaturdifferenzen auf der anderen Seite nur noch minimale Temperaturdifferenzen gemessen werden können, während alle bisherigen Materialien auch bei stärkerer Isolierkraft zu größeren Temperaturschwankungen auf der anderen Seite der Schicht führen. Dies gilt für Isolierstoffe ganz allgemein und alle bisher bekannten Einlegesohlen und Schuhsohlen im besonderen.

Die erfindungsgemäßen Mittel kommen aber auch bei der Anwendung auf anderen Teilen der Außenhaut zur Wirkung. So können derartige Mittel in Form von verschieden gestalteten Pflastern auf die Haut aufgebracht werden und dort ihre angenehme, lindernde oder gar heilende Wirkung entfalten. So lassen sich derartige Pflaster mit Hilfe eines selbstklebenden Pflasters auf dem Körper befestigen. Anstelle des selbstklebenden Pflasters können sie selbstverständlich auch mit Hilfe einer Stoffbinde oder eines Strumpfes befestigt werden. Eine weitere bevorzugte Ausführungsform weist auf der äußeren Schaumstoffschicht zusätzlich einen Klettverschluß auf. Die so ausgestalteten erfindungsgemäßen Mittel lassen sich in besonders einfacher Weise mittels elastischer Binden mit Klettverschluß am Körper befestigen. Schließlich ist es auch möglich, auf die PE-Schaumstoffschicht (1) eine hautverträgliche, selbstklebende Schicht aufzubringen, die durch eine abziehbare Schutzfolie abgedeckt ist. Vor der Benutzung wird die Schutzfolie abgezogen und das Mittel direkt auf die Haut geklebt. Die hautver PE-Schaumstoffshicht (1) ist mit durchblutungsfördernden Substanzen getränken, insbesondere solchen, die sich bereits bei herkömmlichen Heilpflastern bewährt haben. Zu derartigen durchblutungsfördernden Substanzen zählen Substanzen aus Senf, Paprica oder Fructus Capsici, die in Mengen von 0,05 bis 5 Gew.-% in diese Schicht eingebracht werden.

Ein weiterer Vorteil der erfindungsgemäßen miteinander verbundenen Schichten ist die erhöhte Stabilität und Strapazierfähigkeit, so daß die erfindungsgemäßen Mittel nicht mehr so leicht reißen und brechen wie die einzelnen Bestandteile.

Selbstverständlich ist es gewünschtenfalls möglich, daß auch die Mittel dieser Ausführungsform zusätzlich perforiert oder geschlitzt werden, um sie für Feuchtigkeit und Wasserdampf durchlässig zu machen. Die Wärmeisolierung und Verteilung der Wärme bleibt dabei nahezu vollständig erhalten.

In den anliegenden Figuren sind einige bevorzugte Ausführungsformen näher erläutert.

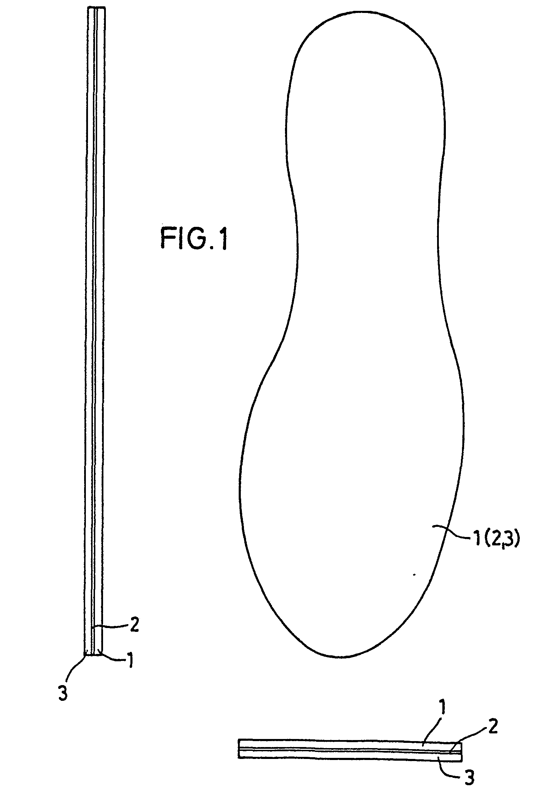

Fig. 1 zeigt eine erfindungsgemäße Einlegesohle;

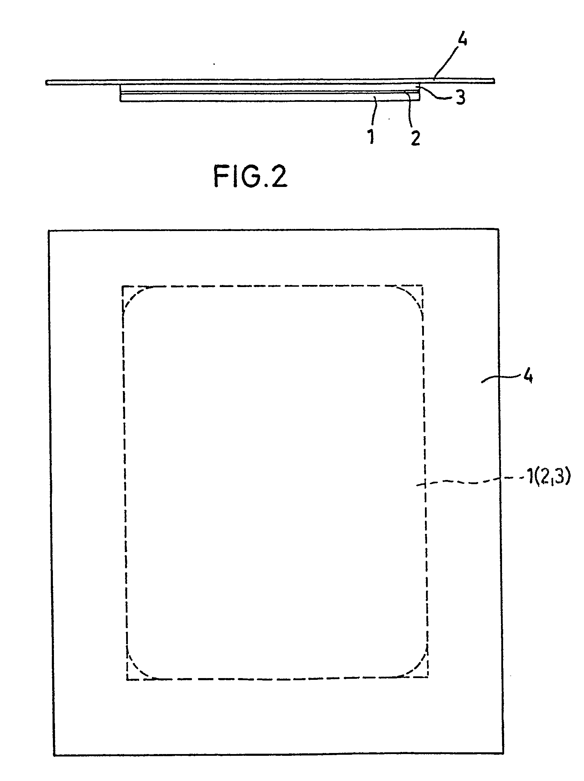

Fig. 2 zeigt ein erfindungsgemäßes Mittel mit einem selbstklebenden Pflaster;

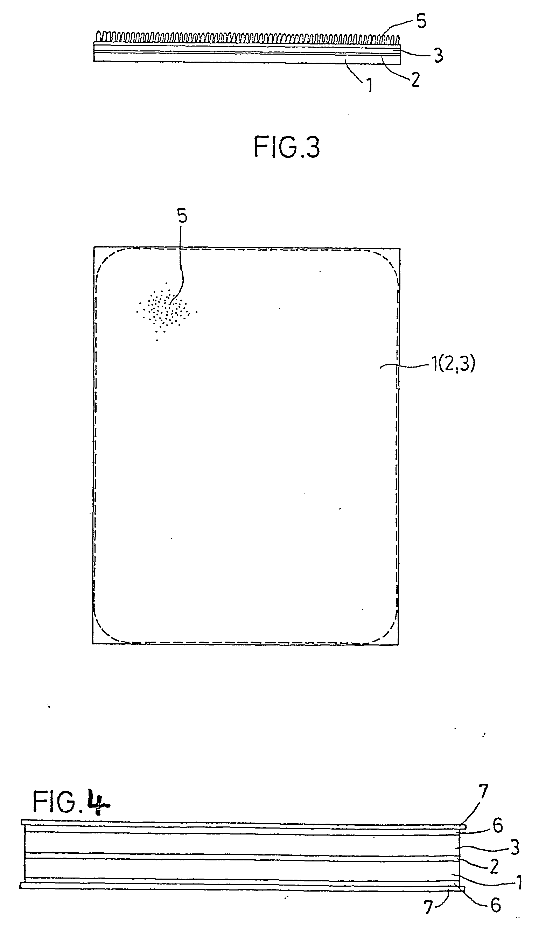

Fig. 3 zeigt ein erfindungsgemäßes Mittel mit einem Klettverschluß und

Fig. 4 den Schnitt durch ein erfindungsgemäßes Mittel, welches auf beiden Seiten hautverträgliche, selbstklebende Schichten und abziehbare Schutzfolien aufweist.

In den Figuren bedeuten jeweils

1) die geschlossenporige PE-Schaumstoffschicht

2) die Foue des Aluminium;

2') eine weiters Folïe aus Aluminium;

3) die weitere geschlossenporige PE-Schaumstoffschicht;

3') eine weitere Schaumstoffschicht aus geschlossenporigem PE-Schaum

4) ein selbstklebendes Pflaster;

5) einen Klettverschluß;

6) eine selbstklebende Schicht;

7) eine abziehbare Schutzfolie.

Temperaturmessungen bei erfindungsgemäßen produkten im Vergleich zu einem Handelsprodukt, bestehend aus einem Textilgewebe, einer Schicht offenporigem Schaum, einer Schicht geschlossenporigem Schaum und einer mit Aluminium bedampften Kunststoffolie haben gezeigt, daß lokale Temperaturschwankungen, ausgehend von 5, 6°C Temperaturdifferenz im Abstand von 2 cm, auf der anderen Seite der Einlegesohle nur noch Temperaturdifferenzen von 0, 1°C zeigten, während bei dem Handelsprodukt noch Temperaturdifferenzen von 0, 7°C gemessen wurden. Im Gegensatz zu der das erfindungsgemäßen Mittel Verwendenden Einlegesohle befindet sich bei dem Handelsprodukt die mit Aluminium bedampfte Kunststoffolie auf der von der Haut abgewandten Seite, während erfindungsgemäß unmittelbar unter der Textilschicht sich die gut wärmeleitende Aluminiumfolie befindet. Die Gesamtdicke der das erfindungsgemäßen Mittel verwendenden Einlegesohle beträgt etwas mehr als 3 mm, während die Dicke des Handelsproduktes 5,5 mm beträgt.

Das Material kann in der oberen und unteren Schaumstoffschicht verschiedenfarbig eingefärbt sein. Sandalen entstehen dadurch, daß im vorderen Teil ein Stoff oder Schaumstoffbügel in passender Größe umgeschlagen und entweder zwischen den beiden Schichten eingeklebt oder seitlich aufgeschweißt bzw. aufgeklebt wird.

**Patentansprüche**

1. Mittel zur Wärmeisolierung sowie zur Speicherung und Verteilung von Körperwärme auf Flächen der Außenhaut bestehend der Reihe nach aus

a) einer 0,8 bis 8 mm starken geschlossenporigen PE-Schaumstoffschicht (1);

b) einer flexiblen, wärmeleitenden Folie aus Aluminium (2) und

c) einer 0,8 bis 8 mm starken geschlossenporigen PE-Schaumstoffschicht (3),

wobei die Schicht (1) mit durchblutungsfördernden Substanzen getränkt ist.

2. Mittel gemäß Anspruch 1, dadurch gekennzeichnet, daß es auf der Schaumstoffschicht (3) einen Klettverschluß (5) aufweist.

3. Mittel gemäß Anspruch 1 oder 2, dadurch gekennzeichnet, daß die der Haut zuzuwendende Seite der PE-Schaumstoffschicht (1) mit einer hautverträglichen Stoffschicht überzogen ist.

**Revendications**

1. Objet destiné à l'isolation thermique et à l'accumulation et la répartition de la chaleur corporelle sur des surfaces de la peau extérieure, composé, dans l'ordre, de

a) une couche de produit alvéolaire en PE à pores fermés d'une épaisseur de 0,8 à 8 mm (1),

b) une feuille flexible, conductrice de la chaleur, en aluminium (2) et

c) une couche de produit alvéolaire en PE à pores fermés d'une épaisseur de 0,8 à 8 mm,

la première couche (1) étant imprégnée de substances favorisant la pénétration.

2. Objet selon la revendication 1, caractérisé en ce qu'il présente une liaison auto-accro-chante, (1) sur la couche de produit alvéolaire (3).

3. Objet selon la revendication 1 ou 2, caractérisé en ce que la face de la couche de produit alvéolaire (1) qui doit être tournée vers la peau est revêtue d'une couche de matière compatible avec celle-ci.

**Claims**

1. Means for insulating heat and for storing and distributing body heat on areas of the outer skin, consisting of, in sequence,

a) a closed-cell polyethylene foam layer (1) having a thickness of from 0.8 to 8 mm;

b) a flexible heat-conductive foil made of aluminum (2); and

c) a closed-cell polyethylene foam layer (3) having a thickness of from 0.8 to 8 mm;

the layer (1) having been impregnated with substances promoting the blood circulation.

2. The means according to claim 1, characterized in that the foam layer (3) has a Velcro strip fastener (5).

3. The means according to claims 1 and 2, characterized in that the surface to be applied such as to face the skin of the polyethylene foam layer (1) has been covered with a skin-compatible layer of a textile material.

FIG.1

1(2,3)

2

1

3

1

2

3

4

3

1 2

# FIG.2

4

-1(2,3)

2

FIG.3

FIG.4